# EUROPEAN PATENT APPLICATION

(11) **EP 4 082 456 A1**
(43) Date of publication of application: **02.11.2022**
(21) Application number: 20905077.2
(22) Date of filing: 24.12.2020
(51) Int. Cl.: A61B 17/3205, A61B 17/42, A61B 17/50

(54) **VIRUS REMOVAL DEVICE AND VIRUS REMOVAL SYSTEM**

(30) Priority: 27.12.2019 JP 2019238222
(71) Applicant: Limited Liability Company Dix, Tokyo 156-0052 (JP)
(72) Inventor: NAMIHIRA Susumu, Tokyo 156-0052 (JP)
(74) Representative: Plasseraud IP
(86) International application number: PCT/JP2020/048600
(87) International publication number: WO 2021/132525

(57) **Abstract**

Viruses around the uterine orifice and in the vagina are reliably and safely removed with a simple configuration. A virus removal device 1 includes a drive source 10 that generates power, a contact part 20 that comes into contact with an affected part around the uterine orifice or in the vagina, a power transmission part 30 that transmits the power generated by the drive source 10 to the contact part 20, and an operation part 40 that operates a position of the contact part 20, in which the contact part 20 is operated by the operation part 40 while being driven by the drive source 10, thereby scraping tissue of the affected part. The contact part 20 scrapes the tissue of the affected part in SCJ, which is a common site of cervical cancer, thereby physically removing the virus contained in the tissue of the affected part to prevent and treat cervical cancer.

## Description

### Technical Field

The present invention relates to a virus removal device and a virus removal system for treating or preventing cervical cancer.

### Background Art

Cervical cancer is a disease almost all of which is associated with human papillomavirus (HPV) infection. HPV causes precancerous lesions in epithelial cells of the cervix and the precancerous lesion develops into cervical cancer. Cervical cancer is a serious disease that affects about 30,000 people per year in Japan, of which about 3000 people die.

The treatment of cervical cancer is generally surgical treatment by resection, radiation treatment, chemotherapy, and the like, and there are a plurality of other treatments such as laser treatment (see, for example, Patent Literature 1), treatment with electromagnetic waves (see, for example, Patent Literature 2), and treatment by injecting a special drug solution into an affected part and irradiating the affected part with light to activate virus eliminating function in the living body (see, for example, Patent Literature 3). These are not only performed independently, but also performed in combination.

### Citation List

### Patent Literature

Patent Literature 1: JP 2015-33504 A
Patent Literature 2: JP 2014-61229 A
Patent Literature 3: JP H10-71212 A

### Summary of Invention

### Technical Problem

However, surgical treatment by resection, radiation treatment, and chemotherapy affect normal cells and tissues together with cancer cells. Then, not only there is a possibility of side effects, but also there is a problem that efficiency of healing is poor. In addition, since the treatment with laser is a treatment on the surface of the epithelium, the efficiency of healing may be poor. Furthermore, a large-scale equipment for generating or controlling laser, microelectromagnetic waves, light, and the like is required for treatment with laser, treatment with microelectromagnetic waves, and treatment by light irradiation after injection of a drug solution. For this reason, a device becomes large.

In addition, in the case of cervical cancer, cells infected with HPV do not become cancer cells immediately, but become cancer cells through a state of dysplasia. Removal of HPV regardless of the result of cytology can effectively prevent cervical cancer.

Therefore, an object of the present invention is to provide a virus removal device and a virus removal system that reliably and safely remove viruses around the uterine orifice and in the vagina with a simple configuration.

### Solution to Problem

A typical configuration of virus removal devices (1, 2, 3, 4, 5, 6, 7, 8) of the present invention includes a drive source (10) that generates power, any of contact parts (20, 21, 22, 23, 24, 25, 26) that come into contact with an affected part around the uterine orifice (U) in the cervix or vagina, any of power transmission parts (30, 34, 37, 38) that transmit the power generated by the drive source to the contact part, and either of operation parts (40, 47) that operate a position of the contact part, in which the contact part is operated by the operation part while being driven by the drive source in order to treat or prevent cervical cancer to scrape tissue of the affected part, thereby removing human papillomaviruses in the tissue. In this way, the contact part comes into contact with the affected part and scrapes the tissue, thereby removing viruses contained in the tissue. This makes it possible to reliably remove viruses around the uterine orifice and in the vagina. On the other hand, since the tissue is regenerated, the possibility of side effects due to treatment is extremely small. This makes it possible to safely remove viruses around the uterine orifice and in the vagina. In addition, since the size of the contact part is small enough to be inserted into the vaginal opening, and the output of the drive source may be small enough to drive the contact part, a large-scale equipment is not required. Therefore, the virus removal device can have a simple configuration. Furthermore, the contact part driven by the drive source removes the tissue of the affected part only by an operator such as a user operating a position of the operation part to contact or press the contact part against the affected part. As a result, it is possible to efficiently scrape the tissue of the affected part as compared with the case of manually moving the contact part to scrape the tissue.

In the virus removal devices (1, 2, 3, 4, 5), the power transmission parts (30, 34, 37) may have long drive shafts (31, 33) driven by the drive source (10), and the contact parts (20, 21) may rotate with rotation of the drive shaft. When the contact part is rotationally driven as described above, the user does not need to perform a rotational operation, and the tissue of the affected part can be efficiently scraped.

In the virus removal devices (1, 2, 3, 4, 5), the contact parts (20, 21) may be configured to be rotatable in both directions of forward rotation and reverse rotation. As a result, the tissue of the affected part can be more efficiently scraped as compared with the case of rotating only in one direction.

In the virus removal devices (1, 2), the contact parts (20, 21) may be formed in a conical shape. When the contact part is formed in a conical shape as described above, a tip of the conical shape easily enters the uterine orifice, and the side surface of the conical contact part effectively comes into contact with the uterine cervical portion around the uterine orifice. HPV, which is a cause of cervical cancer, is likely to infect a squamo-columnar junction (SCJ) around the uterine orifice, and thus the SCJ is a common site of cervical cancer. Therefore, by forming the contact part in a conical shape and bringing the side surface of the contact part into contact with the uterine cervical portion around the uterine orifice, the side surface of the contact part comes into contact with a wide range of the SCJ. As a result, the tissue of the affected part can be efficiently removed.

In the virus removal device (3), the contact part (22) may be configured such that a central portion (22a) is formed in a conical shape, and a peripheral portion (22b) continuously disposed from the central portion is curved in a dish shape. By forming the central portion of the contact part in a conical shape in this way, the tip of the central portion easily enters the uterine orifice. Then, the side surface of the central portion of the conical shape effectively comes into contact with the uterine cervical portion around the uterine orifice. In addition, by curving the peripheral portion of the contact part in a dish shape, the peripheral portion of the contact part comes into contact with a vaginal fornix portion close to the uterine orifice. HPV that causes cervical cancer may also be present in the vaginal fornix portion. Therefore, with the above-described configuration of the contact part, the contact part removes the tissue in a wide range around the uterine orifice and the vagina. As a result, the tissue of the affected part can be more efficiently scraped.

In the virus removal devices (1, 2), the contact surface in contact with the affected part in the contact part (21) may have projections (21a) and recesses (21b), and the projections (21a) or the recesses (21b) may be formed in a lattice shape on the contact surface. With this configuration, the projection (21a) can easily remove the tissue of the affected part as compared with a case where the contact surface is smooth, so that the removal of the tissue of the affected part can be more efficiently performed.

In the virus removal devices (4, 5), the contact parts (23, 24) may be formed of a quadrangular member, and protrusions (23a, 24a) may be formed on a contact surface formed on an outer periphery of the quadrangular member and be in contact with the affected part. As described above, when the contact part is a quadrangular member and the protrusion is provided on the contact surface, the protrusion removes the tissue of the affected part, so that the tissue of the affected part can be efficiently removed.

In the virus removal device (5), the contact part (24) may include a plurality of quadrangular members (24A, 24B). By using a plurality of members in this way, the contact surface of the contact part is widened. As a result, the tissue of the affected part can be efficiently removed.

In the virus removal device (6), the power transmission part (38) may have the long portion (35) and the drive shaft (36) disposed in a direction intersecting the long portion and driven by the drive source (10), and the contact parts (25, 26) may rotate with rotation of the drive shaft. By disposing the contact part around the drive shaft disposed in the direction intersecting the long portion in this way, the contact part efficiently comes into contact with the vaginal fornix portion. In addition, the power of the drive source rotates the contact part, thereby assisting the operation in which the contact part scrapes the tissue. As a result, the tissue of the affected part can be efficiently removed.

In the virus removal device (7), the power transmission part (38) may have the long portion (35) and the drive shaft (36) disposed in a direction intersecting the long portion and driven by the drive source (10), and the contact parts (25, 26) may swing with the swing of the drive shaft. By disposing the contact part around the drive shaft disposed in the direction intersecting the long portion in this way, the contact part efficiently comes into contact with the vaginal fornix portion. In addition, the power of the drive source swings the contact part, thereby assisting the operation in which the contact part scrapes the tissue As a result, the tissue of the affected part can be efficiently removed.

In the virus removal device (8), the power transmission part (38) may have the long portion (35) driven by the drive source (10), and the contact parts (25, 26) may be disposed in a direction intersecting the long portion, and reciprocate between a direction approaching the affected part and a direction away from the affected part, or reciprocate in a direction parallel to a contact surface of the affected part. By disposing the contact part in the direction intersecting the long portion in this way, the contact part efficiently comes into contact with the vaginal fornix portion. In addition, the user does not need to reciprocate the contact part by reciprocating the contact part. Furthermore, if the contact part reciprocates in the direction approaching the affected part and in the direction away from the affected part, viruses inside a tissue hit by the reciprocating motion of the contact part move so as to float on the surface side of the tissue. This makes it easier to remove viruses.

In the virus removal devices (6, 7, 8), the contact parts (25, 26) may be formed of a triangular member. As described above, when the contact parts are formed of a triangular member, the tissue of the affected part can be efficiently removed.

In the virus removal devices (6, 7, 8), a plurality of protrusions (26a) may be formed on the contact surface in contact with the affected part in the contact part (26). By forming the protrusion on the contact surface in this way, the protrusion can efficiently remove the tissue of the affected part.

In the virus removal devices (1, 2, 3, 4, 5, 6, 7, 8), the contact parts (20, 21, 22, 23, 24, 25, 26) may be configured to be replaceable. As a result, since the shape and size of the contact part can be changed according to the affected part, the tissue of the affected part can be efficiently removed. In addition, by replacing the contact part with a clean contact part, sanitary state can be maintained favorably, so that the treatment can be made safe.

In the virus removal devices (1, 2, 3, 4, 5, 6, 7, 8), the contact parts (20, 21, 22, 23, 24, 25, 26) may be made of a soft material. This makes it possible to more safely remove the tissue without damaging the affected part.

The virus removal devices (1, 2, 3, 4, 5, 6, 7, 8) may include a light irradiation part (50) for irradiating the affected part with light. As a result, the light irradiated from the light irradiation part illuminates the affected part, so that visibility in the affected part is enhanced. This makes it possible to more efficiently remove the tissue of the affected part.

In the virus removal devices (1, 2, 3, 4, 5, 6, 7, 8), either of the operation parts (40, 47) may be formed integrally with any of the power transmission parts (30, 34, 37, 38), and have either of grip portions (45, 46) to be gripped by a user. With this configuration, when the user grips the grip portion and moves the operation part, the user can directly operate the contact parts (20, 21, 22, 23, 24, 25, 26) via the power transmission part formed integrally with the operation part.

In the virus removal devices (1, 2, 3, 4, 5, 6, 7, 8), the drive source (10) may be disposed integrally with either of the operation parts (40, 47). With this configuration, it is not necessary to separately carry the drive source, and a simple and highly portable configuration is obtained.

The virus removal devices (1, 2, 3, 4, 5, 6, 7, 8) may include a drive control unit (11) that controls driving of the drive source (10), in which the drive control unit controls driving of the drive source on the basis of an instruction from an operator. With this configuration, it is possible to transmit desired control of the operator to the drive source.

In addition, a configuration of a virus removal system (80) of the present invention includes the above-described virus removal devices (1, 2, 3, 4, 5, 6, 7, 8), a detection part (81) that detects a position of the affected part, and a control part (82) that controls the drive source (10) and the operation parts (40, 47), in which the control part may operate a position of the operation part based on a detection result of the detection part, thereby scraping the tissue of the affected part with the contact parts (20, 21, 22, 23, 24, 25, 26). With this configuration, operation of the operation part can be performed without depending on the user.

Note that the reference signs in parentheses above indicate the reference signs of the corresponding components of the embodiments described later as an example of the present invention.

### Advantageous Effects of Invention

According to the virus removal device of the present invention, it is possible to reliably and safely remove viruses around the uterine orifice and in the vagina with a simple configuration.

### Brief Description of Drawings

Fig. 1 is an overall perspective view of a virus removal device of a first embodiment.
Fig. 2 is an operation explanatory diagram of the virus removal device of the first embodiment.
Fig. 3 is a view illustrating a contact part of a modification of the first embodiment.
Fig. 4 is a view illustrating a grip portion of a modification of the first embodiment.
Fig. 5 is an overall perspective view of a virus removal device of a second embodiment.
Fig. 6 is an operation explanatory diagram of the virus removal device of the second embodiment.
Fig. 7 is an overall perspective view of a virus removal device of a third embodiment.
Fig. 8 is an operation explanatory diagram of the virus removal device of the third embodiment.
Fig. 9 is a view illustrating a modification of the third embodiment.
Fig. 10 is an overall perspective view of a virus removal device of a fourth embodiment.
Fig. 11 is an operation explanatory diagram of the virus removal device of the fourth embodiment.
Fig. 12 is a view illustrating a contact part of a modification of the fourth embodiment.
Fig. 13 is a view illustrating an example in which the contact part swings in the fourth embodiment.
Fig. 14 is a view illustrating an example in which the contact part vibrates in the front-rear direction in the fourth embodiment.
Fig. 15 is an operation explanatory diagram of an example in which the contact part vibrates in the front-rear direction in the fourth embodiment.
Fig. 16 is an operation explanatory diagram of an example in which the contact part vibrates in the left-right direction in the fourth embodiment.
Fig. 17 is a schematic diagram of a virus removal system.

### Description of Embodiments

Hereinafter, preferred embodiments of the virus removal device and the virus removal system of the present invention will be described in detail with reference to the accompanying drawings. In the following description, all embodiments of the virus removal device will be described, and then embodiments of the virus removal system will be described.

In addition, in the following description, a "tissue" refers to all cell tissues that may be infected with HPV around the uterine orifice and in the vagina. That is, the "tissue" in the following description includes a mucous membrane, an epithelial cell, a uterine tissue, and a vaginal tissue.

### First Embodiment

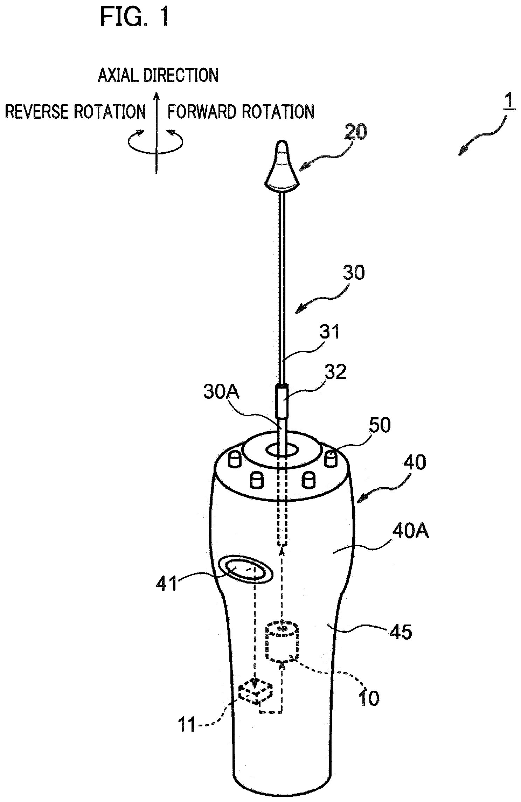

A virus removal device 1 of a first embodiment will be described with reference to the drawings. Fig. 1 is an overall perspective view of the virus removal device of the first embodiment. As shown in Fig. 1, the virus removal device 1 includes a drive source 10 for generating power, a contact part 20 that comes into contact with the affected part, a power transmission part 30 for transmitting the power generated by the drive source 10 to the contact part 20, an operation part 40 for operating a position of the contact part 20, and a light irradiation part 50 for irradiating the affected part with light.

In the present embodiment, in order to reduce the size of the device, the drive source 10 is exemplified by a small motor that can be stored in the operation part 40. However, the configuration of the drive source 10 is not limited thereto, and other means may be used as long as power can be generated.

The contact part 20 is attached to an end portion of the drive shaft 31 opposite to the operation part 40 in the axial direction of the drive shaft 31 (hereinafter, also simply referred to as "axial direction"). The contact part 20 is a member that comes into contact with the affected part around the uterine orifice or in the vagina to scrape the tissue of the affected part. The size of the contact part 20 is such that the contact part can be inserted into the vaginal opening and the vagina.

The material of the contact part 20 is preferably a soft material. This makes it possible to more safely remove the tissue without damaging the affected part. As the soft material, a soft sponge which is a material such as a caring toothbrush is preferable. However, the material of the contact part 20 is not limited thereto, and may be any material as long as the tissue of the affected part can be safely scraped. Therefore, the material of the contact part 20 can be widely selected from resin materials such as silicon and rubber.

The contact part 20 of the present embodiment is formed in a conical shape. Specifically, the contact part 20 is configured such that the size of the contact part 20 in a radial direction orthogonal to the axial direction gradually increases from a tip (an end portion on the opposite side to a direction in which the power transmission part 30 is present in the axial direction) toward the power transmission part 30. The size of the tip of the contact part 20 is such that the tip can enter the uterine orifice. In the present embodiment, the contact part 20 has a conical shape whose diameter continuously increases from the tip toward the power transmission part 30. However, the present embodiment is not limited thereto, and a cylindrical portion having the same diameter may be included in a part of the side surface of the contact part 20.

The power transmission part 30 is a part that transmits the power generated by the drive source 10 to the contact part 20. The power transmission part 30 of the present embodiment has a rotation shaft 30A, a drive shaft 31, and an attachment member 32. The rotation shaft 30A is formed integrally with the operation part 40 and rotates by the power of the drive source 10. The drive shaft 31 is a long shaft to which the power of the rotation shaft 30A is transmitted, and is configured to have at least a length that allows the contact part 20 to reach the uterine orifice. The attachment member 32 is a member for attaching the drive shaft 31 to the rotation shaft 30A. When the rotation shaft 30A and the drive shaft 31 are connected by the attachment member 32, the power of the rotation shaft 30A is transmitted to the drive shaft 31 via the attachment member 32. The attachment member 32 is attachable to and detachable from at least the drive shaft 31. Therefore, the drive shaft 31 and the contact part 20 formed integrally with the drive shaft 31 can be replaced.

In addition, the power transmission part 30 preferably has a structure in which the whole or a part thereof has flexibility. In the case of the present embodiment, the rotation shaft 30A and the drive shaft 31 themselves may be configured to bend, the attachment member 32 may be configured to bend, or both may be configured to bend. As a result, it is possible to effectively remove the tissue by applying appropriate pressure to the affected part, and it is possible to safely remove the tissue by preventing application of excessive pressure to the affected part.

The operation part 40 is a part that is held and moved by an operator such as a user to operate the position of the contact part 20. The operation part 40 of the present embodiment includes a drive instruction unit 41 disposed on the surface of a main body 40A and a grip portion 45 formed on the surface of the main body 40A. The drive instruction unit 41 includes an input unit such as a button or a switch. A grip portion 45 is formed in a columnar shape that is easily gripped by a human hand, and is formed in a size that is easy to operate. Note that the surface of the grip portion 45 may be provided with an uneven shape for preventing slipping, or may be provided with an anti-slip member.

In addition, the operation part 40 of the present embodiment includes the drive source 10 and a drive control unit 11 that controls the drive source 10 inside the main body 40A. The drive source 10 of the present embodiment rotationally drives the rotation shaft 30A disposed in the main body 40A. The drive control unit 11 controls driving of the drive source 10 in accordance with an instruction from the drive instruction unit 41. Specifically, the drive control unit 11 controls rotation speed, rotation direction, and rotation time, in addition to drive start and drive stop of the drive source 10.

The light irradiation part 50 includes an LED light or the like, and is provided on a surface of the operation part 40 on the contact part 20 side. The light irradiation part 50 irradiates at least in the direction of the contact part 20 with light. A plurality of the light irradiation parts 50 of the present embodiment are attached to the operation part 40 at predetermined intervals in the circumferential direction on the circumferential surface around the rotation shaft 30A, and can reliably brighten the affected part. However, the arrangement of the light irradiation parts 50 is not limited thereto, and may be one light source. Furthermore, the light irradiation part 50 may not be an LED light, and may be a light source such as a light bulb.

Note that the light irradiation part 50 of the present embodiment includes only a light source, but is not limited thereto. For example, the light irradiation part 50 may have a reflecting mirror for guiding light in a predetermined direction, in addition to the light source, or may have a configuration for diffusing light.

With this configuration, when the user gives an instruction using the drive instruction unit 41, the drive control unit 11 rotates the rotation shaft 30A using the drive source 10. The rotation of the rotation shaft 30A is transmitted to the drive shaft 31 via the attachment member 32, and is transmitted to the contact part 20 disposed at the tip of the drive shaft 31. Therefore, the contact part 20 rotates around the drive shaft 31 as the central axis. In addition, the drive shaft 31 not only rotates in one direction (forward rotation direction) but also rotates in the other direction (reverse rotation direction). Therefore, the contact part 20 rotates in both the forward rotation direction and the reverse rotation direction. The rotation direction of the contact part 20 is instructed by the operator using the drive instruction unit 41.

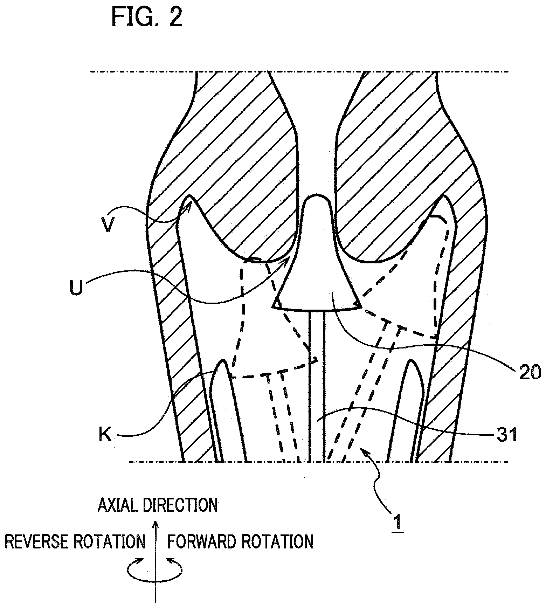

Fig. 2 is an operation explanatory diagram of the virus removal device 1 of the first embodiment. First, the affected part will be described with reference to Fig. 2, and then operation of the virus removal device 1 will be described. HPV, which is a cause of cervical cancer, is likely to infect a squamo-columnar junction (SCJ) around the uterine orifice U, and thus the SCJ is a common site of cervical cancer. Therefore, the uterine orifice U or a vaginal fornix portion V around the uterine orifice U is likely to be an affected part. In the present embodiment, the affected part is a site where a virus causing cervical cancer (HPV) is present.

As shown in Fig. 2, when the virus removal device 1 is used, as a preparation step, a drug having an antiinflammatory effect/antitumor activity effect is supplied to the affected part in a state where the vagina is expanded by colposcope K. A method of supplying the drug is not necessarily limited, but it is preferable to supply the drug by spraying the drug. Thereafter, the contact part 20 at the tip of the drive shaft 31 of the virus removal device 1 is inserted from the vaginal opening. Then, the contact part 20 reaches the uterine orifice U. Here, in accordance with an instruction from the drive control unit 11, the contact part 20 rotates in the forward rotation direction or the reverse rotation direction around the drive shaft 31 as the central axis. Then, when the operator operates the operation part 40 to bring the contact part 20 into contact with the affected part or press the contact part 20 as necessary, the contact part 20 scrapes tissue of the affected part. Then, the virus present in the tissue is physically removed.

In addition, HPV that causes cervical cancer can be present not only in the SCJ present around the uterine orifice U but also in the vaginal fornix portion V. In this case, as shown in Fig. 2, the tissue in the vaginal fornix portion V can be scraped by moving the position of the contact part 20 also to the vaginal fornix portion V as necessary.

As described above, according to the present embodiment, the contact part 20 comes into contact with the affected part and scrapes the tissue, thereby removing viruses contained in the tissue. This makes it possible to reliably remove viruses around the uterine orifice U and in the vagina. On the other hand, since the tissue is regenerated, the possibility of side effects due to treatment is extremely small. This makes it possible to safely remove viruses around the uterine orifice U and in the vagina. In addition, since the size of the contact part 20 is small enough to be inserted into the vaginal opening, and the output of the drive source 10 may be small enough to drive the contact part 20, a large-scale equipment is not required. Therefore, the virus removal device 1 has a simple configuration. Furthermore, the contact part 20 driven by the drive source 10 removes the tissue of the affected part only by the operator operating a position of the operation part 40 to contact or press the contact part 20 against the affected part. As a result, it is possible to efficiently scrape the tissue of the affected part as compared with the case of manually moving the contact part 20 to scrape the tissue.

Further, as in the present embodiment, when the contact part 20 rotates with the rotation of the long drive shaft 31, the user does not need to perform a rotational operation, and the tissue of the affected part can be efficiently scraped. Furthermore, since the contact part 20 is configured to be rotatable in both directions of forward rotation and reverse rotation, the tissue of the affected part can be more efficiently scraped as compared with the case of rotating only in one direction.

In addition, when the contact part 20 has a conical shape as in the present embodiment, a tip portion in the conical shape easily enters the uterine orifice U, and the side surface in the conical shape effectively comes into contact with the uterine cervical portion around the uterine orifice U. Then, the side surface of the contact part 20 comes into contact with a wide range of the SCJ. As a result, the tissue of the affected part can be efficiently removed.

Further, in the present embodiment, the contact part 20 is configured to be replaceable. As a result, since the shape and size of the contact part 20 can be changed according to the affected part, the tissue of the affected part can be efficiently removed. Furthermore, by replacing the contact part 20 with a clean contact part, sanitary state can be maintained favorably, so that the treatment can be made safe.

In addition, when the contact part 20 is made of a soft material as in the present embodiment, the removal of the tissue can be performed more safely without damaging the affected part.

Further, when the light irradiation part 50 for irradiating the affected part with light is provided as in the present embodiment, the light irradiated from the light irradiation part 50 illuminates the affected part, so that visibility in the affected part is enhanced. This makes it possible to more efficiently remove the tissue of the affected part.

Furthermore, as in the present embodiment, the operation part 40 is formed integrally with the power transmission part 30, and has the grip portion 45 to be gripped by the user. With this configuration, when the user grips the grip portion 45 and moves the operation part 40, the user can directly operate the contact part 20 via the power transmission part 30 formed integrally with the operation part 40.

In addition, when the drive source 10 is disposed integrally with the operation part 40 as in the present embodiment, it is not necessary to separately carry the drive source 10, and a simple and highly portable configuration is obtained.

Further, when the drive control unit 11 is provided and the drive control unit 11 controls the driving of the drive source 10 on the basis of an instruction from the operator as in the present embodiment, it is possible to transmit control contents (in the present embodiment, rotation start, rotation stop, rotation speed, rotation direction, rotation time, and the like) of the operator to the drive source. Therefore, the operator can perform desired control on the rotation of the contact part 20 using the drive source 10.

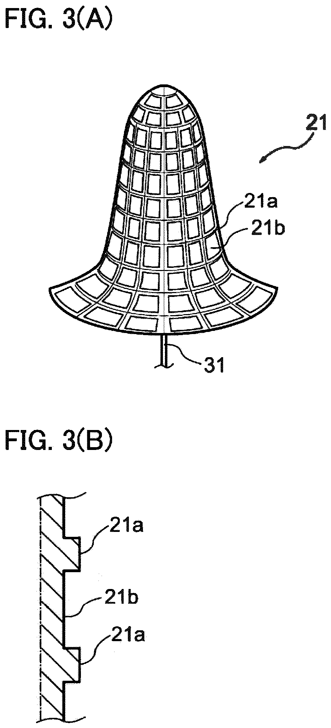

In the present embodiment described above, the configuration of the contact surface of the contact part 20 with the affected part has not been particularly mentioned, but an uneven pattern may be applied to the contact surface of the contact part 20. Fig. 3 is a view illustrating a contact part 21 of a modification of the first embodiment, where (A) is an enlarged perspective view of the contact part 21, and (B) is an enlarged cross-sectional view of uneven parts.

As shown in Fig. 3(A), a lattice pattern including projections 21a and recesses 21b is formed on the side surface of the conical contact part 21. In the present embodiment, as shown in Fig. 3(B), a part projecting in a lattice shape is the projection 21a, and a part recessed as compared with the projection 21a is the recess 21b. The side surface of the contact part 21 is a contact surface in contact with the affected part.

With this configuration, as compared with a case where the contact surface is smooth, the projection 21a is easily elastically deformed, and the projection 21a easily removes the tissue of the affected part. Then, the tissue of the affected part can be more efficiently removed.

Note that the structure of the contact surface is not limited to the present modification. For example, a plurality of projections having a quadrangular prism shape may be formed by forming a lattice pattern into recesses. In addition, the uneven pattern formed on the side surface of the contact part may be other than the lattice pattern.

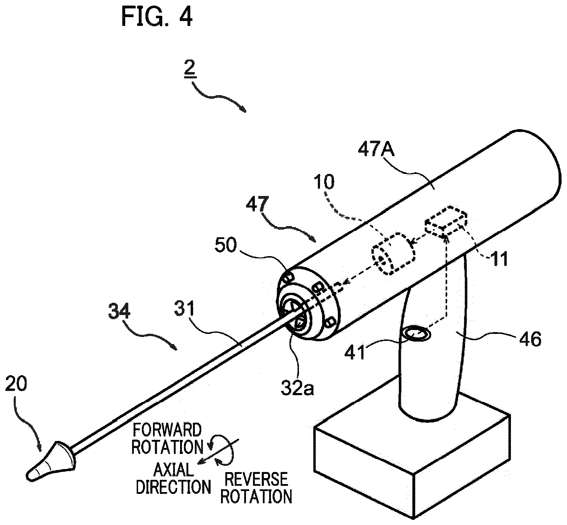

In the present embodiment, the grip portion 45 is formed on the surface of the main body 40A, but is not limited thereto. Fig. 4 is a view illustrating a grip portion 46 of a modification of the first embodiment.

As shown in Fig. 4, the operation part 47 of the virus removal device 2 includes a main body 47A configured to extend in the axial direction of the drive shaft 31, and the grip portion 46 disposed integrally on a side surface of the main body 47A. The grip portion 46 is formed in a columnar shape that can be easily gripped by the user. The longitudinal direction of the columnar shape of the grip portion 46 of the present embodiment is a direction intersecting the axial direction of the drive shaft 31. Here, the intersecting direction is a concept including an orthogonal direction, and is a concept also including a direction inclined with respect to the orthogonal direction to an extent that a member performs a function. The drive instruction unit 41 is disposed in the grip portion 46. As described above, the grip portion 46 may extend from the side surface of the main body 47A of the operation part 47 in the direction intersecting the drive shaft 31.

In addition, the power transmission part 34 of the virus removal device 2 has the drive shaft 31 and an attachment member 32a. The attachment member 32a sandwiches the side surface of the drive shaft 31 from the radially outer side to fix the drive shaft 31 to the main body 47A. Therefore, the drive shaft 31 and the contact part 20 formed integrally with the drive shaft 31 can be replaced.

### Second Embodiment

A virus removal device 3 of a second embodiment will be described with reference to the drawings. The virus removal device 3 is different from the above-described embodiment only in the configuration of the contact part 22. The same reference numerals are given to the same configurations as described above, and the description thereof will be omitted. In addition, description of a configuration having the same action and effect as described above will be omitted. Further, the size, material, and positional relationship of the contact part 22 with respect to the drive shaft 31 are substantially the same as those of the contact part 20 of the first embodiment, and thus the description thereof will be omitted.

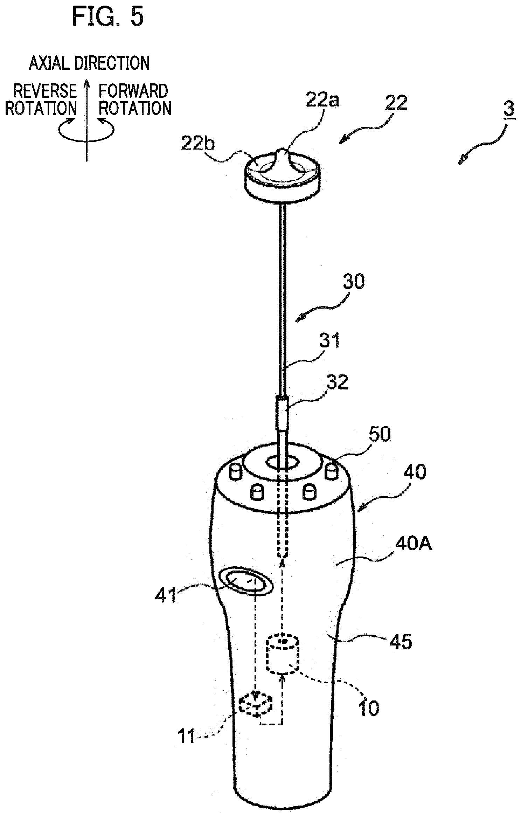

Fig. 5 is an overall perspective view of the virus removal device 3 of the second embodiment. As shown in Fig. 5, the contact part 22 has a conical central portion 22a and a dish-shaped peripheral portion 22b continuously disposed from the central portion 22a.

The central portion 22a is configured such that the size of the central portion 22a in the radial direction orthogonal to the axial direction of the drive shaft 31 gradually increases from a tip toward the power transmission part 30. The size of the tip of the central portion 22a is such that the tip can enter the uterine orifice U. In the present embodiment, the central portion 22a has a conical shape whose diameter continuously increases from the tip toward the power transmission part 30. However, the present embodiment is not limited thereto, and a cylindrical portion having the same diameter may be included in a part of the side surface of the central portion 22a.

The peripheral portion 22b is disposed continuously with an end portion (an end portion on the power transmission part 30 side) of the central portion 22a, and is formed in a dish shape expanding in a radial direction orthogonal to the axial direction. Specifically, the peripheral portion 22b has a dish-like shape that gradually curves toward a tip side of the central portion 22a as it goes radially outward from an inner edge side continuous with the central portion 22a.

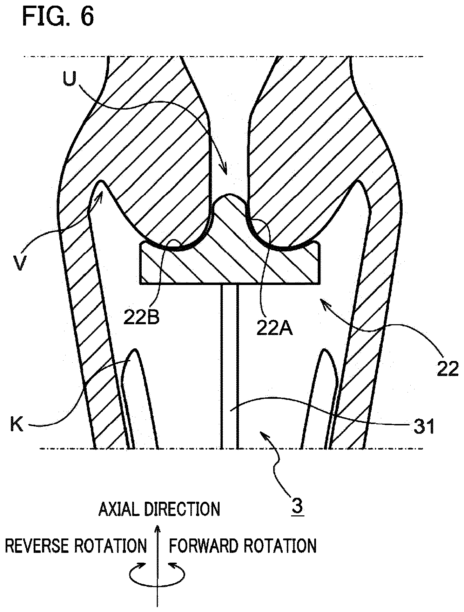

Fig. 6 is an operation explanatory diagram of the virus removal device 3 of the second embodiment. As shown in Fig. 6, when using the virus removal device 3, the contact part 22 at the tip of the drive shaft 31 reaches the uterine orifice U after the procedure of the preparation stage described above is completed. Here, in accordance with instructions (rotation start, rotation stop, rotation speed, rotation direction, rotation time, and the like) from the drive control unit 11, the contact part 22 rotates in the forward rotation direction or the reverse rotation direction around the drive shaft 31 as the central axis. Then, when the operator operates the operation part 40 to bring the contact part 22 into contact with the affected part or press the contact part 22 as necessary, the contact part 22 scrapes the tissue of the affected part.

Here, the tip of the central portion 22a of the contact part 22 enters the uterine orifice U. Therefore, the side surface of the central portion 22a effectively comes into contact with the uterine cervical portion around the uterine orifice U. By forming the central portion 22a in a conical shape in this way, the side surface of the central portion 22a comes into contact with a wide range of the SCJ. In addition, as shown in Fig. 6, the peripheral portion 22b of the contact part 22 comes into contact with the vaginal fornix portion V in the vicinity of the uterine orifice U. As a result, the tissue of the affected part can be efficiently removed in the SCJ and the vaginal fornix portion V.

As described above, according to the present embodiment, the central portion 22a of the contact part 22 is formed in a conical shape. As a result, the tip of the central portion 22a easily enters the uterine orifice U. Then, the side surface of the conical central portion 22a effectively comes into contact with the uterine cervical portion around the uterine orifice U. In addition, by curving the peripheral portion 22b of the contact part 22 in a dish shape, the peripheral portion 22b of the contact part 22 comes into contact with the vaginal fornix portion V close to the uterine orifice U. HPV that causes cervical cancer may also be present in the vaginal fornix portion. Therefore, with the above-described configuration of the contact part 22, the contact part 22 can remove the tissue in a wide range around the uterine orifice U and of the vaginal fornix portion V, and the tissue of the affected part can be more efficiently scraped.

### Third Embodiment

A virus removal device 4 of a third embodiment will be described with reference to the drawings. The virus removal device 4 is different from the above-described embodiment only in the configuration of a contact part 23. The same reference numerals are given to the same configurations as described above, and the description thereof will be omitted. In addition, description of a configuration having the same action and effect as described above will be omitted. Further, the size, material, and positional relationship of the contact part 23 with respect to the drive shaft 31 are substantially the same as those of the contact part 23 of the first embodiment, and thus the description thereof will be omitted.

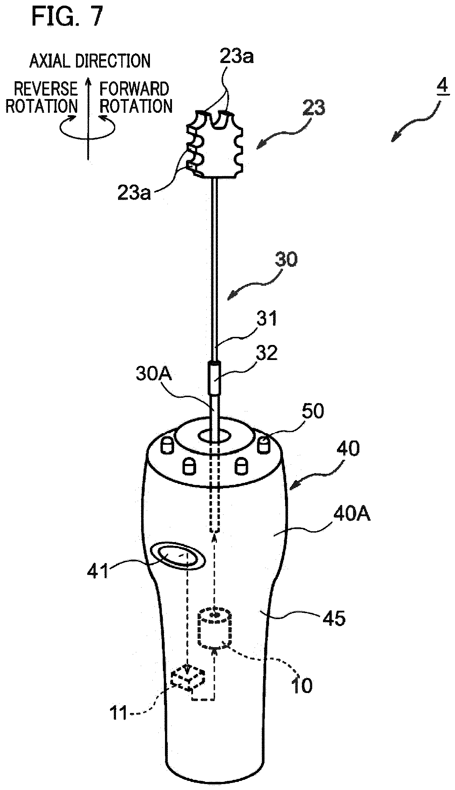

Fig. 7 is an overall perspective view of the virus removal device 4 of the third embodiment. As shown in Fig. 7, the contact part 23 is formed of a quadrangular member, and a plurality of protrusions 23a are formed on a contact surface formed on an outer periphery of the quadrangular member and is in contact with the affected part. In the present embodiment, the protrusions 23a are formed on a total of three sides, a side on a tip side of the contact part 23 and two sides in a direction orthogonal to the axial direction, among four sides of the quadrangular member. In the present embodiment, a plurality of protrusions 23a are formed. Specifically, two protrusions 23a are formed on the side on the tip side, and three protrusions 23a are formed on each of the other two sides. However, the number and size of the protrusions 23a are not limited thereto.

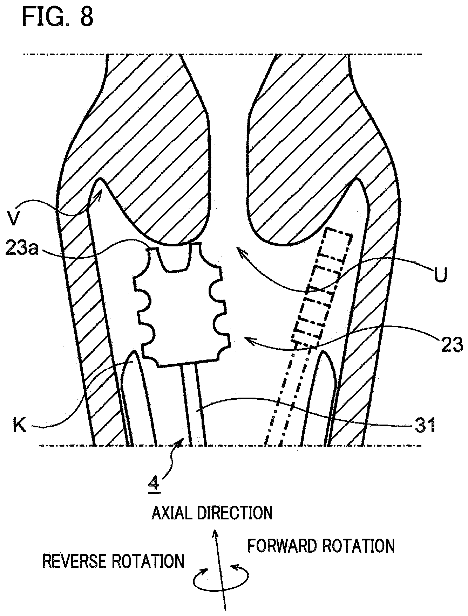

Fig. 8 is an operation explanatory diagram of the virus removal device 4 of the third embodiment. As shown in Fig. 8, when using the virus removal device 4, the contact part 23 at the tip of the drive shaft 31 reaches the uterine orifice U after the procedure of the preparation stage described above is completed. Here, in accordance with instructions (rotation start, rotation stop, rotation speed, rotation direction, rotation time, and the like) from the drive control unit 11, the contact part 23 rotates in the forward rotation direction or the reverse rotation direction around the drive shaft 31 as the central axis. Then, when the operator operates the operation part 40 to bring the contact part 23 into contact with the affected part or press the contact part 23 as necessary, the contact part 23 scrapes the tissue of the affected part. In addition, as shown in Fig. 8, viruses are removed in a wide range by moving the position of the contact part 23 also from around the uterine orifice U to the vaginal fornix portion V.

As described above, according to the present embodiment, when the contact part 23 is a quadrangular member and the protrusion 23a is provided on the contact surface with the affected part, the protrusion 23a effectively removes the tissue of the affected part. In addition, the plurality of protrusions 23a simultaneously remove the tissue of the affected part. Therefore, the tissue of the affected part can be efficiently removed.

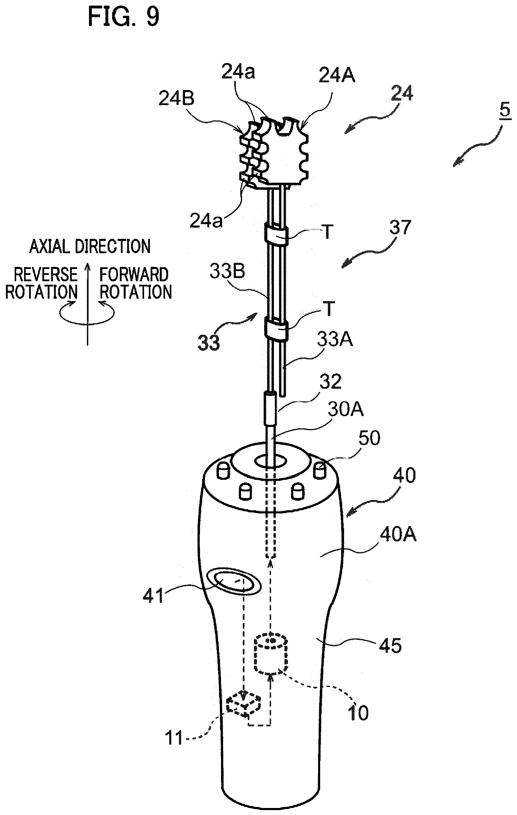

In the above-described present embodiment, the case where the contact part 23 is a single quadrangular member has been exemplified, but a contact part 24 may include a plurality of quadrangular members. Fig. 9 is a diagram illustrating a modification of the third embodiment. The contact part 24 of the virus removal device 5 in the modification includes a first member 24A and a second member 24B each having a quadrangular shape, and each member has a plurality of protrusions 24a.

The configurations of the first member 24A and the second member 24B are similar to the configuration of the contact part 23 described above, and the shape and number of the protrusions 24a are similar to the shape and number of the protrusions 23a, so that the description thereof will be omitted. The first member 24A and the second member 24B are disposed adjacent to each other in a direction intersecting the axial direction. In the present embodiment, the first member 24A is disposed so as to be displaced upward in the drawing in the axial direction with respect to the second member 24B.

As shown in Fig. 9, corresponding to the two members of the contact part 24, the power transmission part 37 has a drive shaft 33 including a first drive shaft 33A and a second drive shaft 33B. A first member 24A is attached to a tip of the first drive shaft 33A, and a second member 24B is attached to a tip of the second drive shaft 33B. The first drive shaft 33A and the second drive shaft 33B are fixed integrally by a binding member T. Of the first drive shaft 33A and the second drive shaft 33B, the second drive shaft 33B is attached to the rotation shaft 30A by an attachment member 32.

When the rotation shaft 30A and the second drive shaft 33B are connected by the attachment member 32, the power of the rotation shaft 30A is transmitted to the first drive shaft 33A and the second drive shaft 33B via the attachment member 32. The attachment member 32 is configured to be attachable to and detachable from at least the second drive shaft 33B. Therefore, the second drive shaft 33B, the second member 24B attached thereto, the first drive shaft 33A fixed to the second drive shaft 33B, and the first member 24A attached to the first drive shaft 33A can be replaced.

As described above, according to the present modification, the contact part 24 includes the first member 24A and the second member 24B that are a plurality of quadrangular members. By using a plurality of members in this way, the contact surface in contact with the affected part is widened. As a result, the tissue of the affected part can be efficiently removed.

### Fourth Embodiment

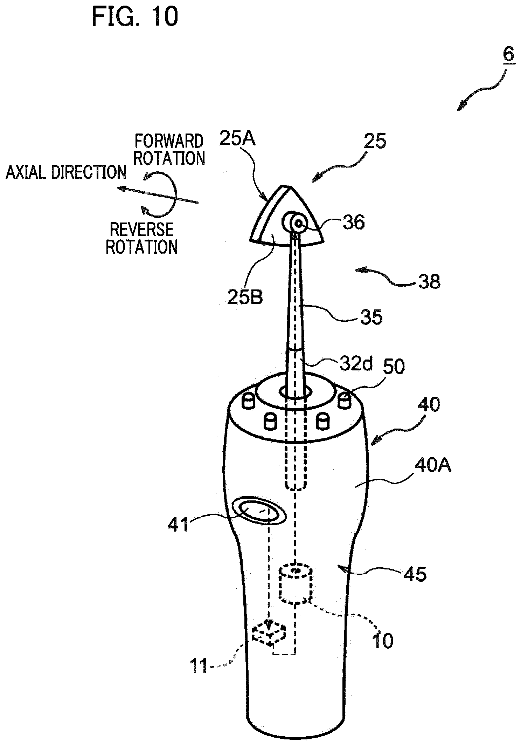

A virus removal device 6 of a fourth embodiment will be described with reference to the drawings. The same reference numerals are given to the same configurations as described above, and the description thereof will be omitted. In addition, description of a configuration having the same action and effect as described above will be omitted. Fig. 10 is an overall perspective view of the virus removal device 6 of the fourth embodiment. As shown in Fig. 10, the virus removal device 6 has a contact part 25 that comes into contact with the affected part, and a power transmission part 38 that transmits the power generated by the drive source 10 to the contact part 20. The configurations of the drive source 10, the operation part 40, and the light irradiation part 50 are similar to those of the above-described embodiment.

The contact part 25 of the present embodiment is formed of a triangular member. In the triangular member, a surface on which a drive shaft 36 to be described later is disposed is referred to as a back surface 25B, and a surface opposite to the back surface 25B is referred to as a front surface 25A. Then, the front surface 25A of the contact part 25 is a contact surface in contact with the affected part.

The power transmission part 38 of the present embodiment includes a long portion 35, a drive shaft 36, and an attachment portion 32d for attaching the long portion 35 to the operation part 40. The long portion 35 is configured to have at least a length that allows the contact part 25 to reach the uterine orifice U. The drive shaft 36 is a rotation shaft disposed at a tip of the long portion 35 and driven by the power transmitted from the drive source 10. The axial direction of the drive shaft 36 is a direction orthogonal to or intersecting the long portion 35. Also, the drive shaft 36 is attached to the back surface 25B of the contact part 25. The attachment portion 32d is a member for forming the long portion 35 integrally with the operation part 40. Therefore, the long portion 35 and the contact part 25 integrated with the long portion 35 via the drive shaft 36 can be replaced.

A mechanism for transmitting the power of the drive source 10 is provided inside the attachment portion 32d and the long portion 35. With this configuration, when the long portion 35 is connected to the attachment portion 32d, the power of the drive source 10 is transmitted to the drive shaft 36.

With this configuration, when the user gives an instruction using the drive instruction unit 41, the drive control unit 11 rotates the drive shaft 36 using the drive source 10. Here, the drive shaft 36 not only rotates in one direction (forward rotation direction) but also rotates in the other direction (reverse rotation direction). Therefore, the contact part 25 rotates in both the forward rotation direction and the reverse rotation direction around the drive shaft 36 as the central axis. The rotation direction of the contact part 25 is instructed by the operator using the drive instruction unit 41.

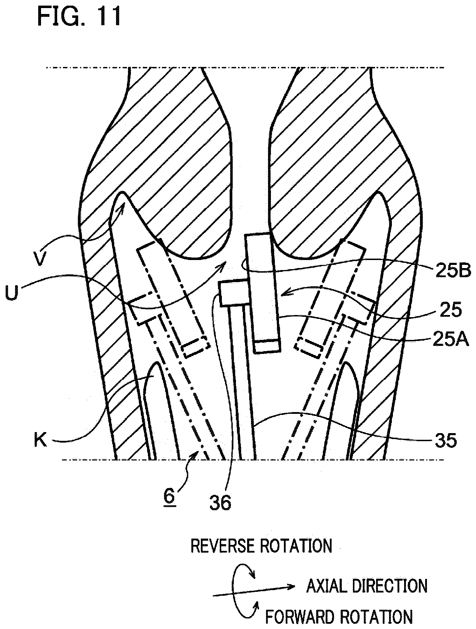

Fig. 11 is an operation explanatory diagram of the virus removal device 6 of the fourth embodiment. As shown in Fig. 11, the contact part 25 at the tip of the drive shaft 36 of the virus removal device 6 is inserted from the vaginal opening after the procedure of the preparation stage described above is completed. Then, the contact part 25 reaches the uterine orifice U or the vaginal fornix portion V around the uterine orifice U. Here, in accordance with instructions (rotation start, rotation stop, rotation speed, rotation direction, rotation time, and the like) from the drive control unit 11, the contact part 25 rotates in the forward rotation direction or the reverse rotation direction around the drive shaft 36 as the central axis. Then, when the operator operates the operation part 40 to bring the front surface 25A of the contact part 25 into contact with the affected part or press the contact part 25 as necessary, the contact part 25 scrapes the tissue of the affected part. Then, the virus present in the tissue is physically removed.

As described above, according to the present embodiment, by disposing the contact part 25 around the drive shaft 36 disposed in the direction intersecting the long portion 35, the contact part 25 efficiently comes into contact with the vaginal fornix portion V. In addition, the power of the drive source 10 rotates the contact part 25, thereby assisting the operation of scraping the tissue by the contact part 25. As a result, the tissue of the affected part can be efficiently removed. Further, when the contact part 25 is formed of a triangular member as in the present embodiment, the tissue of the affected part can be efficiently removed.

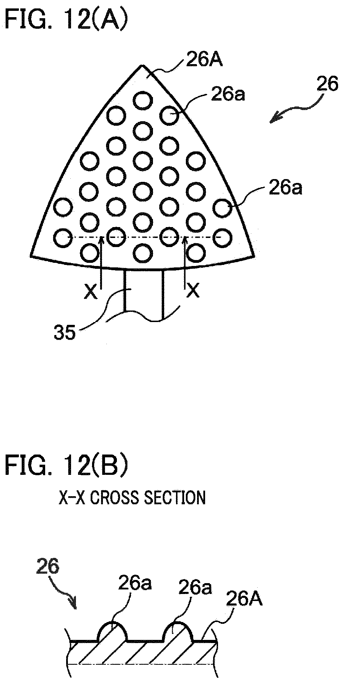

In the present embodiment, the contact surface of the contact part 25 with the affected part does not have a special structure, but the structure of the contact surface is not limited thereto. Next, an example in which a special structure is applied to the contact surface will be described. Fig. 12 is a view illustrating a contact part 26 of a modification of the fourth embodiment, where (A) is a view of the contact part 26 as viewed from a surface 26A, and (B) is a cross-sectional view taken along line X-X in (A) .

As shown in Fig. 12, the contact part 26 may be formed of a triangular member, and a plurality of protrusions 26a may be formed on the surface 26A which is a contact surface of the contact part 26 with the affected part. As shown in Fig. 12(B), the plurality of protrusions 26a of the present modification have a hemispherical shape, and are uniformly arranged on the triangular surface 26A. When the plurality of protrusions 26a are formed on the surface 26A as the contact surface in this way, the plurality of protrusions 26a scrape the tissue of the affected part. As a result, the protrusion 26a can efficiently remove the tissue of the affected part.

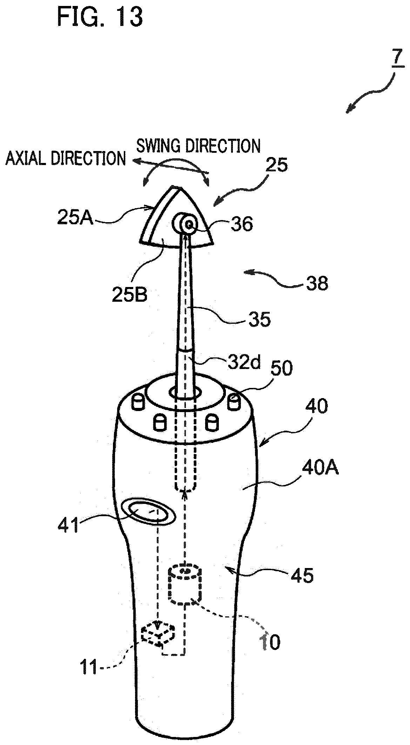

In the present embodiment described above, the contact part 25 rotates by the rotation of the drive shaft 36, but the method of driving the contact part 25 is not limited thereto. Fig. 13 is a view illustrating an example in which the contact part 25 swings in the fourth embodiment. As shown in Fig. 13, in the virus removal device 7, the drive shaft 36 swings, so that the contact part 25 swings about the drive shaft 36 according to instructions (swing start, swing stop, number of swings, swing direction, swing time, and the like) from the drive control unit 11. Then, the contact part 25 assists the operation of scraping the tissue. As a result, the tissue of the affected part can be efficiently removed.

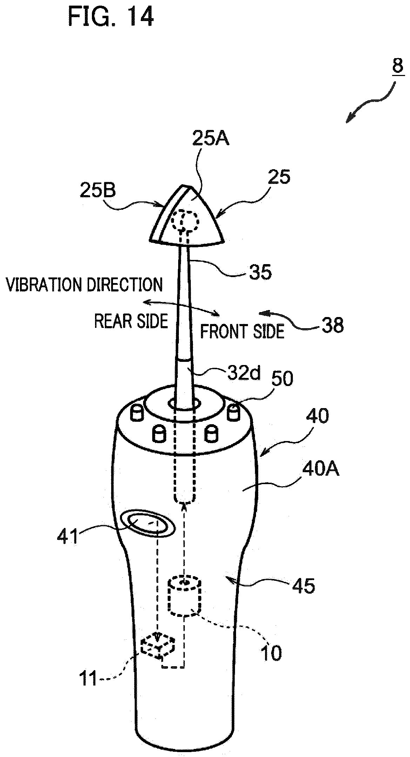

In the present embodiment described above, the drive shaft 36 rotates or swings, but the method of driving the contact part 25 is not limited thereto. For example, the contact part 25 may vibrate (reciprocate) in a predetermined direction. Fig. 14 is a diagram illustrating an example in which the contact part 25 vibrates in the front-rear direction in the fourth embodiment. As shown in Fig. 14, in the virus removal device 8, the power transmission part 38 vibrates in the front-rear direction with respect to the operation part 40. Here, the front-rear direction is a direction of the front surface 25A and the back surface 25B of the contact part 25, and the front surface 25A side of the contact part 25 is defined as a front side and the back surface 25B side of the contact part 25 is defined as a rear side.

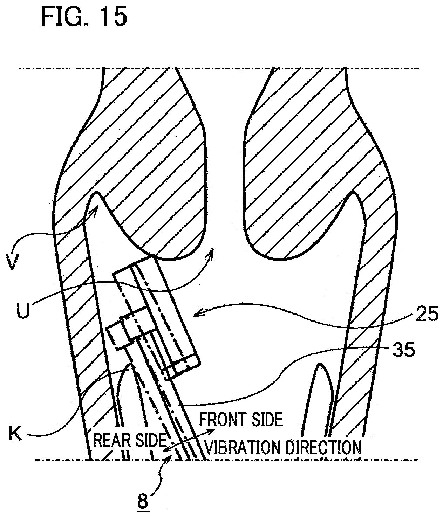

Fig. 15 is an operation explanatory diagram of an example in which the contact part 25 vibrates in the front-rear direction in the fourth embodiment. As shown in Fig. 15, when using the virus removal device 8, the contact part 25 at the tip of the drive shaft 36 of the virus removal device 8 is inserted from the vaginal opening after the procedure of the preparation stage described above is completed. Then, the contact part 25 reaches the uterine orifice U or the vaginal fornix portion V around the uterine orifice U. Here, in accordance with instructions (vibration start, vibration stop, number of vibrations, vibration direction, vibration time, and the like) from the drive control unit 11, the contact part 25 vibrates in the front-rear direction to hit the affected part and remove viruses.

In addition, the contact part 25 reciprocates so as to hit the affected part, whereby the virus inside the tissue moves so as to float on the surface side of the tissue. As a result, the therapeutic effect can be further enhanced. As a result, the tissue of the affected part can be efficiently removed.

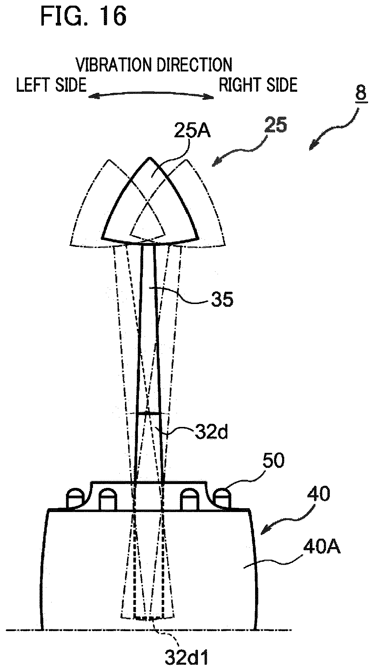

Further, the vibration direction of the contact part 25 of the virus removal device 8 is not limited to only the front-rear direction. Fig. 16 is an operation explanatory diagram of an example in which the contact part 25 vibrates in the left-right direction in the fourth embodiment. As shown in Fig. 16, an end portion 32d1 of the attachment portion 32d opposite to the long portion 35 reciprocates, whereby the contact part 25 disposed at the tip of the long portion vibrates in the left-right direction in accordance with instructions (vibration start, vibration stop, number of vibrations, vibration direction, vibration time, and the like) from the drive control unit 11. Here, the left-right direction is a direction parallel to the contact surface of the affected part in contact with the front surface 25A of the contact part 25. As a result, since the contact part 25 assists the operation of scraping the tissue, the tissue of the affected part can be efficiently removed.

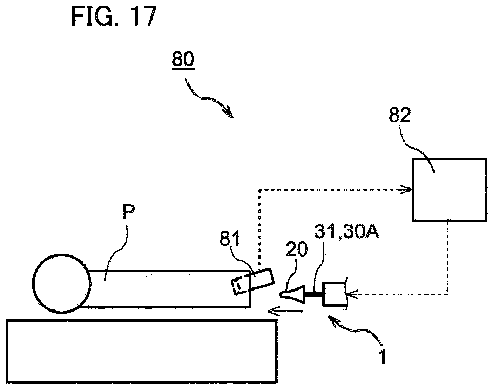

Next, a virus removal system 80 using the above-described virus removal device will be described. Fig. 17 is a schematic diagram of the virus removal system 80. In Fig. 17, an example in which the virus removal device 1 is used for the virus removal system 80 will be described, but a virus removal device according to another embodiment or modification, or a combination thereof may be used.

As shown in Fig. 17, the virus removal system 80 includes a virus removal device 1, a detection part 81, and a control part 82. The detection part 81 detects a position of the affected part. The detection part 81 may grasp the state of the affected part as an image or a video with a camera or the like, or may detect the position of the affected part by another method.

The control part 82 controls drive of the drive source 10 of the virus removal device 1, specifically, controls drive start, drive stop, rotation speed and rotation direction of the drive source 10 via the drive control unit 11. Furthermore, the control part 82 operates the operation part 40 to control a position of the contact part 20.

In this way, the control part 82 operates the position of the operation part 40 on the basis of a detection result of the detection part 81, thereby scraping the tissue of the affected part with the contact part 20. With this configuration, the operation of the operation part 40 can be performed without depending on the user.

### Other Embodiments

Although the preferred embodiments of the present invention have been described above, the present invention is not limited to these embodiments, and various modifications and changes can be made within the scope of the gist of the present invention. In particular, the configurations of the respective embodiments and modifications can be applied to each other within a possible range.

In addition, in the above-described embodiments, the operation of the contact part is only rotation, swinging, and vibration, but the present invention is not limited thereto. For example, the respective operations may be combined. Further, since it is sufficient that the contact part scrapes the tissue of the affected part, an operation of moving the contact part in the axial direction may be added.

Furthermore, in the above-described embodiments, in the case of the configuration in which the drive shaft rotates, it is preferable that the rotation speed of the drive shaft can be changed in a wide range from low-speed rotation to high-speed rotation. For example, the low-speed rotation is about 100 to several hundred rotations per minute, and the high-speed rotation is about 3000 to 3600 rotations per minute. However, the rotation speed of the drive shaft is not necessarily limited thereto, and can be appropriately changed depending on the usage condition of the virus removal device, the condition of the patient, and the like.

Although the embodiments of the present invention have been described above, effects and results associated with the use of these embodiments will be described. The above-described virus removal device has a structure in which the tissue of the affected part is scraped by the contact part. Therefore, the virus removal device of the present embodiment also exhibits effects on treatment of a precancerous lesion state (mild to severe dysplasia) before cervical cancer occurs and mild cervical cancer (for example, stage 1). As described above, the above-described virus removal device is also effective for prevention of cervical cancer.

In addition, since the contact part of the above-described virus removal device can be inserted from the vaginal opening, laparotomy operation or the like is not required. Therefore, it is possible to perform treatment only by visiting a hospital without requiring hospitalization or surgery, and physical burden on the patient is small. Note that, in a case where the above-described virus removal device is used, the virus can be removed by performing treatment for about 10 minutes per time 80 times on average.

Further, the above-described virus removal device does not require large-scale equipment for generating or controlling laser or the like, and is inexpensive, so that economic burden on the patient is small. Furthermore, since the above-described virus removal device is portable and small, it is easy to carry the device. For this reason, prevention and treatment of cervical cancer can be performed even in African area where economy is not rich, cervical cancer frequently occurs, and infrastructure facilities are not advanced.

Next, use results of these embodiments will be described. The virus removal device of the above embodiment can prevent cervical cancer in a normal state or a dysplastic state, and can treat relatively mild stage (around stage I) cervical cancer. As a therapeutic result, among 205 Examples, 205 examples have been completely cured. The test for confirming complete recovery was performed by cytology using Sure Path method liquid-based cytology system. At the same time, the presence or absence of HPV infection is confirmed by performing PCR method three times every ten days, and by no longer detecting HPV (becoming negative), the complete recovery from virus infection is confirmed.

The present application claims priority based on Japanese Patent Application No. 2019-238222 filed on December 27, 2019, the entire contents of which are incorporated herein by reference.

### Reference Signs List

1, 2, 3, 4, 5, 6, 7, 8 Virus removal device
20, 21, 22, 23, 24, 25, 26 Contact part
30, 34, 37, 38 Power transmission part
40, 47 Operation part
50 Light irradiation part
80 Virus removal system

## Claims

1. A virus removal device comprising:
a drive source that generates power;
a contact part that comes into contact with an affected part around the uterine orifice in the cervix or vagina;
a power transmission part that transmits the power generated by the drive source to the contact part; and
an operation part that operates a position of the contact part,
wherein the contact part is operated by the operation part while being driven by the drive source in order to treat or prevent cervical cancer to scrape tissue of the affected part, thereby removing human papillomaviruses in the tissue.

2. The virus removal device according to claim 1, wherein
the power transmission part has a long drive shaft driven by the drive source, and
the contact part rotates with rotation of the drive shaft.

3. The virus removal device according to claim 2, wherein
the contact part is configured to be rotatable in both directions of forward rotation and reverse rotation.

4. The virus removal device according to any one of claims 1 to 3, wherein
the contact part is formed in a conical shape.

5. The virus removal device according to any one of claims 1 to 3, wherein
the contact part is configured such that a central portion is formed in a conical shape, and a peripheral portion continuously disposed from the central portion is curved in a dish shape.

6. The virus removal device according to any one of claims 1 to 5, wherein
a contact surface in contact with the affected part in the contact part has projections and recesses, and
the projections or the recesses are formed in a lattice shape on the contact surface.

7. The virus removal device according to any one of claims 1 to 3, wherein
the contact part is formed of a quadrangular member, and
a protrusion is formed on a contact surface formed on an outer periphery of the quadrangular member and is in contact with the affected part.

8. The virus removal device according to claim 7, wherein
the contact part includes a plurality of the quadrangular members.

9. The virus removal device according to claim 1, wherein
the power transmission part has a long portion and a drive shaft disposed in a direction intersecting the long portion and driven by the drive source, and
the contact part rotates with rotation of the drive shaft.

10. The virus removal device according to claim 1, wherein
the power transmission part has a long portion and a drive shaft disposed in a direction intersecting the long portion and driven by the drive source, and
the contact part swings with swing of the drive shaft.

11. The virus removal device according to claim 1, wherein
the power transmission part has a long portion driven by the drive source, and
the contact part is disposed in a direction intersecting the long portion, and reciprocates between a direction approaching the affected part and a direction away from the affected part.

12. The virus removal device according to claim 1, wherein
the power transmission part has a long portion driven by the drive source, and
the contact part is disposed in a direction intersecting the long portion, and reciprocates in a direction parallel to a contact surface of the affected part.

13. The virus removal device according to any one of claims 9 to 12, wherein
the contact part is formed of a triangular member.

14. The virus removal device according to any one of claims 9 to 13, wherein
a plurality of protrusions are formed on a contact surface in contact with the affected part in the contact part.

15. The virus removal device according to any one of claims 1 to 14, wherein
the contact part is configured to be replaceable.

16. The virus removal device according to any one of claims 1 to 15, wherein
the contact part is made of a soft material.

17. The virus removal device according to any one of claims 1 to 16, comprising:
a light irradiation part that irradiates the affected part with light.

18. The virus removal device according to any one of claims 1 to 17, wherein
the operation part is formed integrally with the power transmission part, and has a grip portion to be gripped by a user.

19. The virus removal device according to any one of claims 1 to 18, wherein
the drive source is disposed integrally with the operation part.

20. The virus removal device according to any one of claims 1 to 19, comprising:
a drive control unit that controls driving of the drive source,
wherein the drive control unit controls driving of the drive source on the basis of an instruction from an operator.

21. A virus removal system comprising:
a virus removal device according to any one of claims 1 to 20;
a detection part that detects a position of the affected part; and
a control part that controls the drive source and the operation part,
wherein the control part operates a position of the operation part based on a detection result of the detection part, thereby scraping tissue of the affected part with the contact part.
